(19) Europäisches Patentamt
European Patent Office
Office européen des brevets

(11) Publication number: **0 514 494 B1**

(12) **EUROPEAN PATENT SPECIFICATION**

(45) Date of publication of patent specification: **28.06.95**

(51) Int. Cl.⁶: **C12Q 1/00**, G01N 33/569, C12Q 1/04, C12Q 1/06

(21) Application number: **91906234.9**

(22) Date of filing: **11.02.91**

(86) International application number:
**PCT/US91/00956**

(87) International publication number:
**WO 91/12337 (22.08.91 91/19)**

(54) **METHOD FOR DETECTION OF HUMAN IMMUNODEFICIENCY VIRUS AND CELL LINES USEFUL THEREFOR.**

(30) Priority: **09.02.90 US 478081**

(43) Date of publication of application:
**25.11.92 Bulletin 92/48**

(45) Publication of the grant of the patent:
**28.06.95 Bulletin 95/26**

(84) Designated Contracting States:
**AT BE CH DE DK ES FR GB GR IT LI LU NL SE**

(56) References cited:
**US-A- 4 704 357**

**JOURNAL OF INFECTIOUS DISEASES vol. 163, 1991, CHICAGO IL USA pages 64 - 70 B. CHESEBRO ET AL. 'Use of a new CD-4 positive HeLa cell clone for direct quantitation of infectious human immunodeficiency virus from blood cells of aids patients.'**

(73) Proprietor: **THE UNITED STATES OF AMERICA as represented by the Secretary UNITED STATES DEPARTMENT OF COMMERCE**

**Washington, DC 20002 (US)**

(72) Inventor: **CHESEBRO, Bruce, Wilcox**
**779 Willow Creek Road**
**Corvallis, MT 59828 (US)**
Inventor: **WEHRLY, Kathryn, Ann**
**512 Bailey Avenue**
**Hamilton, MT 59840 (US)**

(74) Representative: **Jump, Timothy John Simon et al**
**Venner Shipley & Co.**
**20 Little Britain**
**London EC1A 7DH (GB)**

JOURNAL OF VIROLOGY vol. 65, no. 11, 1 November 1991, WASHINTON DC USA pages 5782 - 5789 B. CHESEBRO ET AL. 'Identification of human immunodeficiency virus envelope gene sequences influencing viral entry into CD-4 positive HeLa cells, T-leukemia cells and macrophages.'

Journal of Virology, Vol. 62, No. 10, issued October, 1988, CHESEBRO et al., "Development of a Sensitive Ouantitative Focal Assay for Immunodeficiency Virus Infectivity", pages 3779-3788, see entire reference.

Science, 229, No. 4713, issued 09 August 1985, HARADA, et al., "Infection of HTLV-III/LAV in HTLV-I Carrying Cells MT-2 and MT-4 and Application in a Plaque Assay", pages 563-566, see especially pages 565-566.

European Journal of Immunology, Vol. 16, issued 1986, CHANH, et al., "Human immunodeficiency Virus, gp 120 glycoprotein detected by a monoclonal antibody to synthetic peptide", pages 1465-1468, see especially the Abstract and page 1468.

Virology, Vol. 158, issued 1987, HARADA et al., "Clonal Selection of Human Immunodeficiency Virus (HIV): Serological Differences in the Envelope Antigens of the Cloned Viruses and HIV Prototypes (HTLV-III B, LAV, and ARV)", pages 447-451, see Abstract.

Journal of Medical Virology, Vol. 27, issued 1989, MASUDA et al., "Emergence of Large Plaque-Producing Clones of Human Immunodeficiency Virus (HIV) In Vitro", pages 170-177, see entire reference.

Journal of Virological Methods, Vol. 18, issued 1987, HARADA et al., "Clonal Analysis of functional differences among strains of human immunodeficiency virus (HIV)", pages 291-304, see entire reference.

Journal of Virological Methods, Vol. 26, issued 1989, NAKASHIMA, et al., "Tetrazolium-based plaque assay for HIV-1 and HIV-2, and its use in the evaluation of antiviral compounds", pages 319-330, see entire reference.

Journal of General Virology, Vol. 70, issued 1989, MCKEATING et al., "Evaluation of Human and Simian Immunodeficiency Virus Plaque and Neutralization Assays", pages 3327-3333, see entire reference.

Virology, Vol. 141, issued 1985, SITBOON et al., "Use of Focal Immunofluorescence Assay on Live Cells for Ouantitation of Retroviruses: Distinction of Host Range Classes in Virus Mixtures and Biological Cloning of Dual-Tropic Murine Leukemia Viruses", pages 110-118, see especially pages 110-113 and page 116.

**Description**

The present invention relates to a method for AIDS diagnosis and monitoring of anti-AIDS drug therapy, and more particularly to a method of assaying for human immunodeficiency virus.

AIDS is a relatively newly recognized disease. To date, thousands of cases have been reported, and the disease has elicited great concern because of the rapid expansion of the epidemic and the high mortality rate or the disease. Its overwhelming prevalence among homosexual men with multiple sexual partners, users of illegal intravenous drugs, hemophiliacs, blood transfusion recipients, and close heterosexual contacts of members of the above high-risk groups strongly suggests that the disease spreads by the transmission of an infectious agent. The primary targets of affliction in the human body are specific subpopulations of T-cells. The severe immune deficiency of patients with AIDS results from an unusually low proportion of helper T-cells (T4 cells) in their lymphocyte population, thus reducing the availability of many T4 helper functions, among which is the production of antibodies by B-cells.

Retrovirus infection is known to lead to depressed immune function in animal systems. Analogizing the human response to these non-animal systems, a human retrovirus with a tropism for T-cells was considered a candidate in the etiology of human AIDS.

Several members of a family of human T-lymphotropic retroviruses (HTLV) have been isolated. One of these isolates was obtained from a patient with an aggressive form of T-cell lymphoma. This virus, designated as HTLV-1, has been etiologically linked to the pathogenesis adult T-cell leukemia/lymphoma (ATLL). In vitro infection with HTLV-I can alter T-cell function and, in some cases, lead to T-cell death. Another member of the HTLV-II family was isolated from a patient with a T-cell variant of hairy cell leukemia, and was designated HTLV-II. Isolation of the HTLV-I and HTLV-II have been reported from cultured T-cells of patients with AIDS. Isolation of another retrovirus was reported from a homosexual patient with chronic generalized lymphadenopathy, a syndrome that often precedes AIDS and therefore is referred to as "pre-AIDS". Proviral DNA of HTLV-I was detected in the cellular DNA of two AIDS patients, and sera of some patients were shown to react with antigens of HTLV-I. The correlation between AIDS and serum antibodies to HTLV-I protein is weak.

It has now been shown that the primary cause of AIDS is a human T-lymphocytic retrovirus variant with limited cross-reactivities with the known HTLV subgroups. These new variants are designated HTLV-III, or, more recently, HIV.

Since AIDS and human immunodeficiency virus (HIV) are known to be transmitted by blood products, a method for detecting contamination of blood by HIV is needed to guarantee the safety of recipients of blood. Such a test method should be as sensitive and specific as possible, and should also be easily performed in a routine environment. Tests for viral markers such as reverse transcriptase, viral antigens, or nucleic acid sequences in fresh or cultured blood cells are slow and are presently not suitable for large-scale screening. On the other hand, serological tests for viral antibodies or antigens are well suited for this purpose, since HIV infected individuals are generally seropositive for antibodies to one or more HIV proteins.

Quantitative measurement of HIV, the agent of acquired immune deficiency syndrome, is currently a technically difficult procedure. Primary isolation of HIV from patients is usually done in PHA- or allogenically-stimulated human lymphocyte blast cell cultures. This procedure was subsequently adapted using end-point dilution to give a quantitative (ID50) estimate of infectious virus. However, for many studies, an enumeration, or "plaque", assay would be highly desirable.

A plaque test has been described in HTLV-I-carrying cell lines by Harada et al., in J. Clin. Microbiol. 25, 1305 (1987). This assay is quantitative and useful for the LAV/HTLV IIIB strain of HIV, but it is less sensitive than the use of normal human lymphocyte blast cultures and does not work on wild-type HIV isolates. Furthermore, viruses recovered from such cells have the potential to be altered by recombination with HTLV-I-derived gene sequences, thus obscuring the true genetic structure of the original input HIV strain.

Previous work with other retroviruses has indicated that assay of virus-infected cells by infectious center assay on susceptible monolayers of cells adherent to plastic dishes is a highly sensitive quantitative detection method. Furthermore, focal areas of virus infection can be identified using virus-specific monoclonal or polyclonal antibodies, thus eliminating the requirement for a focal cytopathic effect at each site of infection, which can be difficult or impossible to obtain in certain virus-cell combination. Finally, if detection is done in living cells, biologically cloned viruses can be directly obtained from heterogeneous populations. Such an approach has not been possible with HIV because of the lack of suitable plastic-adherent target cells.

Gallo et al., in U.S. Patent 4,520,113, disclose a method for detecting antibodies to AIDS in sera by either a strip radioimmunoassay based on the Western Blot technique, or ELISA, an enzyme-linked immunosorbent assay.

Currently the standard test for the presence of live HIV in human blood, tissue, or fluid samples involves incubation of samples to be tested together with activated T-lymphocytes obtained from normal human blood. Subsequently, these T-lymphocytes are cultured continuously for up to five weeks, and the culture fluid is examined periodically for the presence of reverse transcriptase enzyme found within HIV virus particles. This culture isolation procedure is not quantitative, and its sensitivity is unclear.

Focal immuno assay (FIA) is a known assay technique, but it has not been used to assay for HIV-infected cells or HIV in the absence of cells because of the lack of appropriate target cells, specific anti-HIV antibodies, and the requisite procedural details to make the test work for HIV.

Chesebro et al. have reported that a quantitative focal assay can be used to detect human immunodeficiency virus infectivity using an adherent cell line expressing the CD4 gene (J. Virol, Vol. 62, No. 10, 3779, 1988).

Most cell lines used for laboratory HIV infection studies grow as suspension cultures in liquid medium. In this case, rare infected cells float around in the cultures, spreading infection randomly. This can only be detected when the level of infected cells has spread enough to be detected by immunofluorescence or reverse transcriptase analysis. This usually takes from about two to four weeks for low levels of initial infection.

It is an object of the present invention to overcome deficiencies in the prior art, such as those noted above. It is another object of the present invention to assay for HIV-infected cells.

It is a further object of the present invention to test for cell-free HIV.

It is still another object of the present invention to provide an improved, more accurate, simplified and faster test for diagnosing AIDS.

It is yet another object of the present invention to monitor levels of HIV or HIV-infected cells in samples obtained from humans.

It is yet a further object of the present invention to monitor levels of HIV or HIV-infected cells in samples produced in laboratory situations.

It is yet another object of the present invention to provide a method for preparing an anti-HIV monoclonal antibody.

It is still a further object of the present invention to select a susceptible target cell for use in an assay for HIV.

Accordingly, in a first aspect of the present invention, there is provided a cell line denoted as number 1022 and deposited in the American Type Culture Collection under accession number CRL-10284, or a functionally equivalent homologue thereto.

In a second aspect of the present invention, there is provided a method of quantitatively assaying for the presence of human immunodeficiency virus in a sample, comprising: seeding dishes with cells obtained from an assay cell line; adding the sample to be assayed to said dishes; incubating the cells; exposing said cells to a first antibody reactive with human immunodeficiency virus proteins; exposing said cells to a marker-conjugated second antibody reactive with said first antibody; and counting the number of foci of marker-positive cells per dish, characterized by the use of an assay cell line as claimed in claim 1.

Preferred embodiments of the invention in either its first or second aspect are as defined in the sub-claims.

The assay according to the present invention is a focal immuno assay (FIA) which uses HIV-specific antibodies and indirect immunological assays such as immunocuto-chemistry or autoradiography immunofluorescence, to detect local areas, or foci, of HIV infection in susceptible target cells growing in monolayers.

The test according to the present invention is both quantitative and highly sensitive. On laboratory HIV strains a single HIV-infected cell can be detected in a sample containing one million uninfected cells, and higher percentages of infected cells can be accurately quantitated. This procedure allows monitoring of the effects of drug therapy on levels of HIV expression in AIDS patients, as well as providing a quantitative measure of HIV expression in asymptomatic HIV antibody-positive individuals. The test according to the present invention is rapid, requiring only two to four days to complete, which is a major advantage compared to presently available tests.

The test according to the present invention is more sensitive for HIV-infected cells than for cell-free HIV. Nevertheless, the test does work with cell-free virus, and can be used to measure virus neutralizing antibody more quantitatively, since individual foci of virus infection can be counted.

To test the efficiency of the present invention, various dilutions of HIV or HIV-infected cells were placed into dishes previously seeded with cells susceptible to HIV infection. After incubation, the cells were exposed to antibody reactive with HIV proteins or a monoclonal antibody for the gap 120 envelope protein

EP 0 514 494 B1

of the LAV/HTLV IIIB strain of HIV, exposed to marker-conjugated antibody reactive with the first antibody used, and incubated. After short term culturing, the dishes were then examined by appropriate means such as a fluorescent microscope or a light-microscope, and the number of foci or immunofluorescent-positive cells per dish were counted.

A process according to the present invention to test for the presence and/or extent of HIV infection involves the steps set forth above, except for addition of HIV or HIV- infected cells.

To insert the appropriate target cells, HeLa cells were infected with a retroviral vector containing the CD4 gene and neomycin resistant gene. After infection, the cells were grown in the presence of the neomycin analog, G418, and resistant clones were selected. Of eighteen clones analyzed, four were found to express cell surface CD4 detectable by membrane immunofluorescence using the monoclonal antibody OKTY.

The present invention uses a focal immunoassay (FIA) which uses HIV-specific antibodies and indirect immunoassay techniques to detect local areas or HIV infection in susceptible target cells growing in monolayers on plastic dishes.

Varying dilutions of the sample to be tested, whether or not unknown or known to contain HIV or HIV-infected cells, are placed into dishes seeded one day previously with cells susceptible to HIV infection. Any suitable growth medium may be used, such as those selected from known growth media. Selection of cells susceptible to HIV infection is important, although not limited to any single type of cells. However, the susceptible target cell must be one which will grow attached to the testing dishes, preferably formed of plastic. Suitable target cells are mentioned in the examples below.

Two to four days later growth medium is removed, and cells are fixed, for example by methanol or other suitable fixers for five to ten minutes. After rinsing of the fixed cells, e.g., with conventional aqueous buffer, the rinsed fixed cells are exposed to an appropriate dilution and volume of antibody reactive with HIV proteins. Again, the selection of anti-HIV antibody is an important consideration, and it is necessary to select the anti-HIV antibody from among those which are capable of detecting some HIV strains in fixed or live infected cells with a high degree of sensitivity. Suitable materials are mentioned in the examples.

After exposure, for thirty minutes, it is desirable to again wash with an aqueous buffer. The so-treated cells are then exposed to marker-conjugated antibody reactive with the first antibody. The marker-conjugated antibody may be conjugated with any suitable marker material such as fluorescein isothiocyanate (FITC) which may be purchased from commercial sources, although clearly other materials can be used. Optimally, a supplemental protein such as bovine serum albumin (BSA) or fetal calf serum (FCS) can be used to enhance responsiveness of the cells.

After further incubation, e.g., thirty minutes, the dishes are again washed, e.g., twice with aqueous buffer. Next, the cells are either fixed, e.g., with methanol or 2% formalin or reacted with suitable chemicals to visualize conjugated antibodies. After optional further washing and removal of fluid, the dishes are subjected to examination in a fluorescence or light microscope or autoradiograph, depending upon the type of marker used.

It was necessary to develop a susceptible target cell for use in the assay of the present invention. A susceptible target cell was developed which would grow attached to plastic dishes. In order to accomplish this, the CD4 gene encoding the HIV receptor molecule, T4, was transferred to a human carcinoma cell line, HeLa. Eighteen different T4-positive HeLa cell clones were derived and screened for HIV infection. Many were negative, and among the positive clones different levels of HIV infectibility were seen. The best clones were chosen for further experiments.

Infection was carried out by placing various dilutions of HIV or HIV-infected cells into dishes which had been seeded one day previously with cells which are susceptible to HIV infection. Two to four days after infection, the growth medium was removed, and the cells were fixed by exposure to methanol for from about five to about ten minutes. After fixation, the cells were rinsed twice with aqueous buffer, exposed for about thirty minutes to an appropriate dilution and volume of mouse or human antibody reactive with HIV proteins, washed once with aqueous buffer, and exposed to a marker-conjugated antibody reactive with the first antibody used.

The anti-HIV antibody was selected as follows. Initially, several mouse monoclonal antibodies specific for the HIV envelope protein were generated, gpl20, which were capable of detecting some HIV strains in methanol-fixed or live infected cells with high sensitivity. These monoclonal antibodies were used in the initial development of procedural details of the FIA and selection of the best target cell clone. However, because it was feared that these antibodies might not detect wild type HIV strains as well as human AIDS patient serum, human AIDS patient serum was also used for HIV detection in the FIA. Initially, this was a problem, since AIDS patient serum contains many antibodies other than anti-HIV. However, by extensive absorption of serum with uninfected human lymphocytes and HeLa cells, non-specific reactivity could be

5

EP 0 514 494 B1

eliminated and absorbed human AIDS patient sera were effective in the test. This monoclonal reacts with the gp 120 protein but does not block the CP 14. The gp 120's can be tagged without interfering with the other reaction.

Monoclonal antibodies reactive with the envelope protein of HIV were generated, ATCC number CRL 9631. Two different antibodies, designated as 902 and 907, were obtained, which antibodies immunoprecipitated the gp 160 and/or gp 120 envelopes from HIV-infected cells or virions, and were shown to be specific for gp 120 by their reactivity with cells infected with vaccinia virus encoding HIV gp 120.

The second step in the detection method according to the present invention uses marker-conjugated antibodies reactive with the human or mouse immunoglobulins used in the first step. These marker-conjugated antibodies can be purchased from commercial sources, such as Cappel Laboratories, in West Chester, PA.

After another incubation of about thirty minutes, the dishes were washed twice again with buffer and fixed again with methanol in 2% formalin or processed to develop marker-specific staining. After one additional aqueous wash, fluid was removed, and the dishes were examined in a suitable detection means, microscope such as a fluorescence microscope or light microscope of an autoradiograph.

The number of foci of marker-positive cells per dish were then counted. Usually these foci consist of from about 1 to about 30 cells in a small area sometimes containing one or more cells with multiple nuclei (about 2 to more than 30). Since from about one to about five million lymphocytes or monocytes can be placed in a single dish, and one can detect a single focus in a dish, the assay can detect approximately one infected cell per one to five million cells tested.

Other variables which have been dealt with to make the assay of the present invention successful include the density of the target seeding ($5 \times 10^4$ per 35 mm dish), number of days between infection and focus detection (2 to 4 days), type of fixation, and method of observation.

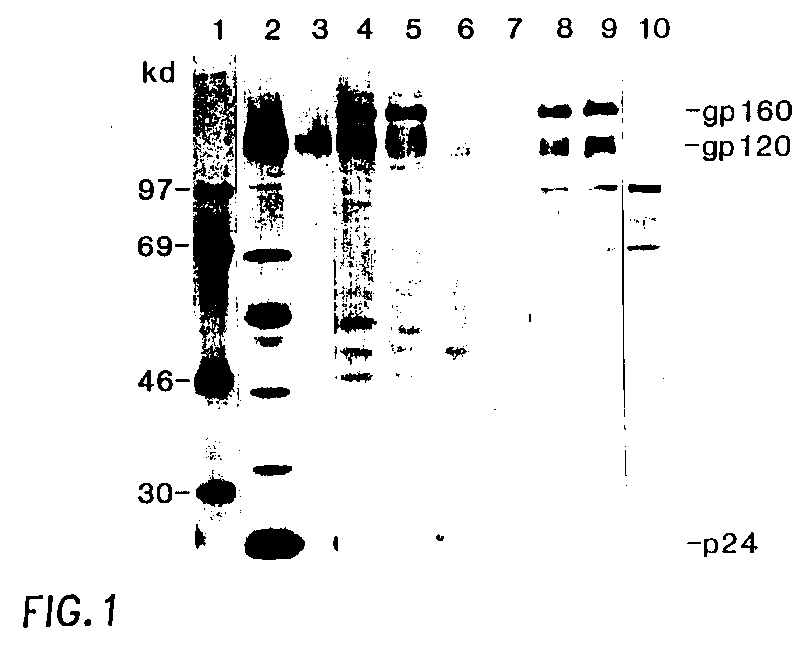

The present inventors have previously found that monoclonal antibodies specific for retroviral envelope proteins were very useful as specific immunological reagents to detect live or fixed virus-infected cells. To facilitate development of an HIV assay, monoclonal antibodies were generated which were reactive with the envelope protein of HIV. Two different antibodies, designated 902 and 907, were obtained which immunoprecipitated the gp160 and/or gp120 envelope proteins from HIV-infected cells or virions, as shown in Figure 1, and were shown to be specific for gp120 by their reactivity with cells infected with vaccinia virus encoding HIV gp120.

The cause of the relative inefficiency of initial infection of conventional HT4-6C cells (ATCC accession number CRL-9631) by wild-type HIV virions is unknown (see Chesebro et al. J. Virol, Vol. 62, No. 10, 3779, 1988). This may be due to an insufficient quantity of T4 receptor molecules expressed on these cells. In this regard, 18 other HeLa clones infected with the CD4 expression vector were tested, and a large variation in sensitivity of HIV detection was found. Alternatively, it is also possible that other receptor molecules besides T4, and even membrane lipid composition, might contribute to more efficient HIV infection.

A new cell line recently discovered which has been designated as 1022 cell line has now been used in the method of the invention. The cell line is 10X to 330X more sensitive than the HT4-6C cell line at detecting wild-type HIV strains directly after isolation from AIDS patients. The cells of Maddon and the HT4-6C cell line are compared with the 1022 cell line of the present invention. While Maddon suggests many of the aspects of the currant method, the cells of Maddon are not reliable for purposes of the invention, since the foci of the HIV infection are difficult to observe due to the roughness of the monolayer and the number of foci detected is very low. Furthermore, the method of this invention using the cell lines of the invention permit quantitative evaluation of viral particles in the sample, since the parameter measured is the focus of infection, each focus being generated by a single original infectious HIV particle.

Table 1 is provided comparing the prior art cell lines with the cell line and the techniques of the invention.

6

Table [1]  Comparison of human immunodeficiency virus infectivity titers on three different CD4-positive HeLa cell lines.

| Virus | Madden | HIV foci/0.2 ml 6C | 1022 | Ratio 1 |
|---|---|---|---|---|
| 13539 | <3 | 57 | 19000 | 3 |
| 13948 | 24 | 48 | 1410 | |
| 13956 | 29 | 512 | 33100 | |
| 14114 | 15 | 100 | 6020 | |
| 208K#2 | 13 | 141 | 30200 | 2 |
| 208K#6 | 38 | 195 | 20800 | 1 |
| 208K#9 | 8 | 214 | 23000 | 1 |
| 208K#10 | <3 | 19 | 4570 | 2 |
| 14345 | 10 | 26 | 3020 | 1 |
| 14558 | 17 | 246 | 16600 | |
| 212#1 | 6 | 126 | 1350 | |
| NL4-3 | 204 | 214 | 692 | |

NL4-3 is a laboratory adapted HIV clone which grows in many human T cell leukemi. All other viruses are HIV isolates from AIDS patients which were passaged 1-2 times in human PHA-stimulated lymphocytes and do not grow in human T cell leukemia lines. Patient isolates not adapted to growth in leukemia cell lines, displayed titers in 1022 cells which were 333-fold higher than in 6C cells. Titers in Madden cells were lower than in 6C cells.

None of the patient's lymphocytes tested were positive when analyzed directly by FIA prior to in vitro cultivation. Therefore, based on the number of cells tested, these patient cell populations contained less than one FIA-positive cell per million cells. However, in many cases, co-cultivation in vitro for several days in the presence of normal PHA blasts led to amplification of HIV expression to levels detectable by FIA. In most instances, detection by FIA slightly preceded the appearance of reverse transcriptase. However, this kinetic difference was usually small under these assay conditions.

Chesebro et al., Virology 84, 222 (1978) have already found that detection of retrovirus-infected cells by infectivity assays is highly dependent on the rate of infectious virus release by individual cells. Cells releasing virus slowly are detected inefficiently, whereas cells releasing virus rapidly are readily detected. This situation appears also to hold true for cells infected with HIV. Previous studies of peripheral blood mononuclear cells from AIDS patients indicated that cells containing HIV gene sequences could be detected by in situ hybridization at a frequency of around 1 per $10^5$ cells in half of the patients. If the rate of virus production per cell were sufficiently high, such a frequency should be easily within the sensitivity of detection of these cells by FIA. The finding that FIA assays of cells taken directly from patients were consistently negative (i.e., less than one positive cell per $10^6$ cells) but became positive after in vitro co-cultivation, suggested that the rate of virus release was very low in patient peripheral blood cells but was elevated during in vitro cultivation. This conclusion agrees with estimates of very low HIV gene copy numbers observed by in situ hybridization of AIDS patient blood cells. Low virus expression is not usually seen in retroviral diseases, however, central nervous system "latent" infection by visna lentivirus is associated with the presence of a very low number of viral genes and low levels of viral protein and infectivity in infected cells. In this disease, the mechanism of regulation of expression is unknown, but the low expression is believed to be important in long-term virus persistence in vivo. Interestingly, similar to the results with HIV, low expression of visna virus was reversed when cells were cultured in vitro.

To obtain the results shown in Figure 1, HIV (LAV strain)-infected A3.01 cells were labelled with $^{35}$S-cysteine at 120 microcuries/ml and 2 a $10^6$ cells/ml for eight hours at 37°C in cysteine-free medium with dialyzed fetal calf serum NIH/3T3 cells infected with vaccinia expressing HIV gp160 or vaccinia expressing influenza (HA) (18) at a multiplicity of 10, and twenty hours later cells were labelled with $^{35}$S-methionine at 100 microcuries/ml for two hours at 37°C in methionine-free medium.

After labelling, the cells were precleared and processed from immunoprecipitation using 9 x $10^5$ cell equivalents of lysate of 0.10 ml of NP40-lysed culture supernatant fluid incubated with 1 ml of hybridoma tissue culture supernatant of 3 microliters human AIDS patient serum. Then, 0.1 ml of a 10% suspension of Sepharose ®protein A (Pharmacia) was added, incubated for thirty minutes with gentle rocking, and the beads were then washed three times with lysing buffer, eluted and analyzed by SDS-PAGE in 8% polyacrylamide gels followed by fluorography as described by Chesebro et al., Virology 112, 131 (1981).

In Lane 1, molecular weight markers are shown. Lane 2 shows 2, human anti-HIV AIDS patient serum with supernatant of HIV-infected A3.01 cells. Lane 3 shows monoclonal antibody 902 with supernatant of HIV-infected A3.01 cells. Lane 4 shows human anti-HIV with lysate of HIV-infected A3.01 cells. Lane 5 shows monoclonal antibody 902 with lysate of HIV-infected A3.01 cells. Lane 6 shows human anti-HIV with lysate of uninfected A3.01 cells. Lane 7 shows monoclonal antibody 902 with lysate of uninfected A3.01 cells. Lane 8 shows monoclonal antibody 902 with lysate of Vaccinia-HIV gp160-infected NIH/3T3 cells. Lane 10 shows monoclonal antibody 902 with lysate of vaccinia-influenza HA-infected NIH/3T3 cells. The locations of the HIV envelope proteins, gp160 and gp120, and the major HIV core protein, p24, are shown on the right.

Hybridomas producing monoclonal antibodies specific for HIV envelope proteins were obtained from spleen cells of (BIO.A (2R) X A.BY)F mice immunized with a x $10^7$ PFU of vaccinia virus expressing HIV gp160 (1B) first in a tail scratch and three to five weeks later intraperitoneally. Three days after the second inoculation, spleen cells were fused with NSI cells.

Supernatant fluids were screened by indirect immunofluorescence on methanol-fixed 8E5 cells which chronically express HIV envelope and gag proteins. Six positive clones were obtained from one mouse, and they appeared to represent at least two distinct clones (antibodies 902 and 907) based on differences in L chain migration in SDS-PAGE gels. All antibodies were of IgG1 subclass, and in lysing buffer, all bound to Sepharose protein A, but not to heat and formalin-fixed staph A (Cowan I strain). All antibodies were specific for HIV gp120 based on reactivity in indirect immunofluorescence with methanol-fixed CV-1 cells infected with vaccinia expressing HIV gp160 or gp120. No reactivity was observed against CV-1 cells infected with vaccinia expressing influenza HA. Furthermore, these antibodies reacted in indirect immunofluorescence with live and methanol-fixed A3.01 cells and MOLT-4 cells infected with HIV (LAV strain), H9 cells infected with HIV (HTLV IIIB strain), and 8E5 cells, but were nonreactive with uninfected A3.01, MOLT4, and H9 cells, or with A3.01 cells or PHA blast cells infected with all other HIV isolates tested so far.

The effects of drug therapy are monitored in patients suffering from AIDS by taking serum samples from the patients and testing for the presence of HIV-infected cells according to the above-described procedure. A decrease in the number of HIV-infected cells during the course of therapy is a positive indication of the efficacy of the drug against the disease.

EXAMPLE II

HIV expression can be quantified in asymptomatic HIV antibody-positive patients by testing for the presence of HIV in the serum of these patients according to the procedure of the present invention.

EXAMPLE III

The virus neutralizing antibody in serum is measured using the assay procedure as described above.

Undoubtedly the present invention for determining presence of HIV virus grows more important as more anti-AIDS drugs become available and as tests for viral resistance to the drug which is being used on a particular patient become desirable. By the method of the invention, HIV-susceptible cells are attached to a solid substrate and exposed to the test material to be investigated for presence of HIV virus. The assay as method for detecting drug-resistant HIV strains in those undergoing treatment for HIV infection will become increasingly important as more therapeutic options are available to the practitioner. (See Larder et al., "HIV with Reduced Sensitivity to Zidovidine (AZT) Isolated During Prolonged Therapy" Science, Vol. 243, pp 1731-1734). The method provides a means of determining the number of infectious HIV particles in a sample by counting the foci of HIV-positive cells in a field. HIV positive cells are detected by the presence of HIV proteins or nucleic acids in individual cells using appropriate antibodies or probes, or by the typical HIV-induced changes in cell morphology such as fusion and syncytia formation. The progress of the disease as measured by the number of HIV virus in a sample.

For detecting inhibitor drug-resistant HIV strains, the cells are infected with HIV and incubated for 3 days in the presence of different drug concentration. The HIV foci are then counted. The concentration of drug required to reduce the number of foci by 50% or 95% is then calculated. Because the method provides a means of identifying foci, the method is quantitative, and can be used as a method to evaluate sensitivity of a particular patient's viral strain to various therapeutic agents. Hence, it provides a means for the physician to choose the particular anti-AIDS drug which is most likely to be effective for inhibiting the strain of HIV infecting an individual.

The techniques could also be used to screen new drugs of efficacy against HIV.

The cell line 1022 (ATCC accession No, CRL-10284) was deposited on February 8, 1988 in the American Type Culture Collection (ATCC), 12301 Parklawn Drive, Rockville, Maryland, 20852, U.S.A. under the provisions of the Budapest Treaty. The deposit will be maintained in accord with that treaty. After the patent is granted, the deposited cell line will be accessible to the public.

The deposit will be stored with care necessary to keep it viable and uncontaminated for a period of at least five years after the most recent request for furnishing a sample of the cell line and, in any case, at least thirty (30) years after the date of the deposit or for the enforceable life of the patent, whichever is longer.

**Claims**

1. A human Hela cell line expressing the CD4 antigen, denoted as number 1022 and deposited in the American Type Culture Collection under accession number CRL-10284, or a functionally equivalent homologue thereto.

2. A method of quantitatively assaying for the presence of human immunodeficiency virus in a sample, comprising: seeding dishes with cells obtained from an assay cell line; adding the sample to be assayed to said dishes; incubating the cells; exposing said cells to a first antibody reactive with human immunodeficiency virus proteins; exposing said cells to a marker-conjugated second antibody reactive with said first antibody; and counting the number of foci of marker-positive cells per dish, characterized by the use of an assay cell line as claimed in claim 1.

3. A method as claimed in claim 2, wherein the number of cells infected with human immunodeficiency virus is determined from the number of foci of marker-positive cells per dish.

4. A method as claimed in claim 3, wherein the antibody reactive with said first antibody is conjugated with a detectable marker.

5. A method as claimed in claim 4, wherein the marker is fluorescein isothiocyanate.

6. A method as claimed in claim 4, wherein the marker is horseradish peroxidase.

7. A method as claimed in claim 3, wherein the first antibody is a monoclonal antibody.

8. A method as claimed in claim 3, wherein the first antibody is reactive with the envelope protein of HIV.

9. A method as claimed in claim 3, wherein the time between seeding of the dishes and counting of the foci is from about two to about four days.

10. A method as claimed in claim 3, wherein the cells are fixed after the incubation step.

11. A method as claimed in claim 10, wherein the cells are fixed with a compound selected from the group consisting of methanol and formaldehyde.

12. A method as claimed in claim 2, wherein the sample comprises cells and said sample is added to the dishes by seeding the dishes with said cells to detect if said cells are infected with human immunodeficiency virus.

13. A method as claimed in claim 12, comprising monitoring the effects of drug therapy on an AIDS patient by assaying sample cells from an AIDS patient to determine if the drug is affecting the cells of the AIDS patient.

14. A method as claimed in claim 12, comprising quantifying HIV expression in sample cells from an HIV antibody-positive patient.

**15.** A method as claimed in claim 12, comprising assaying a body fluid for human immunodeficiency virus by using sample cells from said body fluid in the assay.

**16.** A method as claimed in claim 15, wherein said body fluid is selected from the group consisting of blood, semen and urine.

**Patentansprüche**

**1.** Eine menschliche, das CD4-Antigen ausprägende HeLa-Zellinie, die durch die Nummer 1022 bezeichnet und in der American Type Culture Collection unter der Zugriffsnummer CRL-10284 hinterlegt ist, oder ein funktionell äquivalentes Homolog davon.

**2.** Ein Verfahren zur quantitativen Prüfung auf das Vorhandensein von Human Immunodeficiency Virus in einer Probe, umfassend: Beimpfung von Schalen mit von einer Prüfzellinie gewonnenen Zellen; Zusatz der zu prüfenden Probe zu den besagten Schalen; Inkubation der Zellen; Aussetzung der besagten Zellen einem ersten mit Proteinen des Human Immunodeficiency Virus reaktionsfähigen Antikörper; Aussetzung der besagten Zellen einem zweiten mit Markierstoff konjugierten Antikörper, der mit dem besagten ersten Antikörper reaktionsfähig ist; und Zählen der Anzahl von Herden markierstoffpositiver Zellen je Schale, gekennzeichnet durch die Verwendung einer Prüfzellinie nach Anspruch 1.

**3.** Ein Verfahren nach Anspruch 2, bei dem die Anzahl der mit Human Immunodeficiency Virus infizierten Zellen aufgrund der Anzahl von Herden markierstoffpositiver Zellen je Schale bestimmt wird.

**4.** Ein Verfahren nach Anspruch 3, bei dem der mit dem besagten ersten Antikörper reaktionsfähige Antikörper mit einem nachweisbaren Markierstoff konjugiert ist.

**5.** Ein Verfahren nach Anspruch 4, bei dem der Markierstoff Fluoresceinisothiocyanat ist.

**6.** Ein Verfahren nach Anspruch 4, bei dem der Markierstoff Meerrettichperoxidase ist.

**7.** Ein Verfahren nach Anspruch 3, bei dem der erste Antikörper ein monoklonaler Antikörper ist.

**8.** Ein Verfahren nach Anspruch 3, bei dem der erste Antikörper mit dem Füllprotein des HIV reaktionsfähig ist.

**9.** Ein Verfahren nach Anspruch 3, bei dem die Zeit zwischen dem Beimpfen der Schalen und dem Zählen der Herde von etwa zwei Tagen bis etwa 4 Tagen beträgt.

**10.** Ein Verfahren nach Anspruch 3, bei dem Zellen nach der Inkubationsstufe fixiert werden.

**11.** Ein Verfahren nach Anspruch 10, bei dem die Zellen mit einer aus der Methanol und Formaldehyd umfassenden Gruppe ausgewählten Verbindung fixiert werden.

**12.** Ein Verfahren nach Anspruch 2, bei dem die Probe Zellen umfaßt und die besagte Probe durch Beimpfung der Schalen mit den besagten Zellen zu den Schalen zugesetzt wird, um zu bestimmen, ob die besagten Zellen mit dem Human Immunodeficiency Virus infiziert sind.

**13.** Ein Verfahren nach Anspruch 12, umfassend Überwachung der Auswirkungen von Drogentherapie auf einen AIDS-Patienten durch Prüfen von Probezellen, die von einem AIDS-Patienten gewonnen wurden, um zu bestimmen, ob die Droge auf die Zellen des AIDS- Patienten eine Wirkung ausübt.

**14.** Ein Verfahren nach Anspruch 12, umfassend quantitative Bestimmung des Grades der HIV-Ausprägung in von einem HIV-Antikörperpositiven Patienten erhaltenen Probezellen.

**15.** Ein Verfahren nach Anspruch 12, umfassend die Prüfung einer Körperflüssigkeit auf Human Immunodeficiency Virus durch Verwendung von aus der besagten Körperflüssigkeit stammenden Probezellen in der Prüfung.

**16.** Ein Verfahren nach Anspruch 15, bei dem die besagte Körperflüssigkeit aus einer Blut, Samen und Harn umfassenden Gruppe ausgewählt wird.

**Revendications**

**1.** Lignée cellulaire humaine de HeLa, exprimant l'antigène CD4, identifiée par le numéro 1022 et déposée auprès de l'American Type Culture Collection sous le numéro d'accès CRL-10284, ou une lignée homologue fonctionnellement équivalente à ladite lignée.

**2.** Procédé pour analyser de manière quantitative la présence du virus de l'immunodéficience humaine, dans un échantillon, comprenant les étapes suivantes :

ensemencement de boîtes avec des cellules obtenues à partir de la lignée cellulaire d'essai;

on additionne l'échantillon destiné à être testé aux dites boîtes;

incubation des cellules;

exposition desdites cellules avec un premier anticorps réagissant avec les protéines du virus de l'immunodéficience humaine;

exposition desdites cellules à un second anticorps conjugué à un marqueur, ledit anticorps réagissant avec ledit premier anticorps; et

comptage du nombre de foyers de cellules marquées par boîtes, caractérisée en ce qu'on utilise une lignée cellulaire d'essai telle que revendiquée à la revendication 1.

**3.** Procédé selon la revendication 2, selon lequel le nombre de cellules infectées par le virus de l'immunodéficience humaine est déterminé à partir du nombre de foyers de cellules marquées par plaque.

**4.** Procédé selon la revendication 3, selon lequel l'anticorps réagissant avec ledit premier anticorps est conjugué à un marqueur détectable.

**5.** Procédé selon la revendication 4, selon lequel le marqueur est l'isothiocyanate de fluorescéine.

**6.** Procédé selon la revendication 4, selon lequel le marqueur est la peroxidase de raifort.

**7.** Procédé selon la revendication 3, selon lequel le premier anticorps est un anticorps monoclonal.

**8.** Procédé selon la revendication 3, selon lequel le premier anticorps réagit avec la protéine d'enveloppe du VIH.

**9.** Procédé selon la revendication 3, selon lequel la période entre l'ensemencement des boîtes et le comptage des foyers est d'environ deux à environ quatre jours.

**10.** Procédé selon la revendication 3, selon lequel les cellules sont fixées après l'étape d'incubation.

**11.** Procédé selon la revendication 10, selon lequel les cellules sont fixées par un composé choisi dans le groupe consistant en le méthanol et le formaldéhyde.

**12.** Procédé selon la revendication 2, selon lequel l'échantillon comprend des cellules, et ledit échantillon est additionné aux boîtes en ensemençant les boîtes avec lesdites cellules à détecter, si lesdites cellules sont infectées par le virus de l'immunodéficience humaine.

**13.** Procédé selon la revendication 12, comprenant l'étape de contrôle des effets de la thérapie médicamenteuse sur un patient atteint de SIDA, en analysant les cellules de l'échantillon d'un patient atteint de SIDA, pour déterminer si le médicament affecte les cellules dudit patient.

**14.** Procédé selon la revendication 12, comprenant l'étape de quantification de l'expression du VIH dans les cellules de l'échantillon d'un patient ayant des anticorps anti-VIH.

**15.** Procédé selon la revendication 12, comprenant l'étape d'analyse d'un fluide corporel vis-à-vis de la présence du virus de l'immunodéficience humaine, en utilisant des cellules de l'échantillon prélevé à partir dudit fluide biologique, au cours du test.

16. Procédé selon la revendication 15, selon lequel ledit fluide corporel est choisi dans le groupe consistant en le sang, le sperme et l'urine.

1 2 3 4 5 6 7 8 9 10

kd

-gp160
-gp120

97-

69-

46-

30-

-p24

*FIG.1*